# EUROPEAN PATENT APPLICATION

(11) **EP 4 603 511 A1**
(43) Date of publication of application: **20.08.2025**
(21) Application number: 23877326.1
(22) Date of filing: 12.10.2023
(51) Int. Cl.: C07K 16/40, A61K 39/395, A61P 35/00

(54) **ANTIBODY AND USE THEREOF**

(30) Priority: 14.10.2022 JP 2022165631
(71) Applicant: JSR Corporation, Tokyo 105-8640 (JP); Kyoto University, Kyoto-shi, Kyoto 606-8501 (JP)
(72) Inventor: ISHIKAWA, Hidefumi, Tokyo 105-8640 (JP); KANAHARA, Masaaki, Tokyo 105-8640 (JP); MIZUUCHI, Motoaki, Tokyo 105-8640 (JP); YAMAGUCHI, Tetsuji, Tokyo 105-8640 (JP); MATSUZAWA, Shuichi, Kyoto-shi, Kyoto 606-8501 (JP)
(74) Representative: TBK
(86) International application number: PCT/JP2023/036979
(87) International publication number: WO 2024/080325

(57) **Abstract**

An antibody or a fragment thereof, in which complementarity determining regions (CDR-H1 to CDR-H3) of a heavy chain variable region (V_{H} region) include each of amino acid sequences set forth in SEQ ID NOs: 1 to 3, and complementarity determining regions (CDR-L1 to CDR-L3) of a light chain variable region (V_{L} region) include each of amino acid sequences set forth in SEQ ID NOs: 4 to 6.

## Description

### TECHNICAL FIELD

The present invention relates to an antibody and use thereof. Priority is claimed on Japanese Patent Application No. 2022-165631, filed October 14, 2022, the content of which is incorporated herein by reference.

### BACKGROUND ART

A protein tyrosine kinase 7 (PTK7), which is also known as a colon carcinoma kinase 4 (CCK-4), is known to be overexpressed in various diseases including cancer. Therefore, it is considered that the inhibition of the signal transduction mediated by PTK7 may lead to the development of a therapeutic method for a disease.

For example, it is known that PTK7 and a degradation product thereof control the motility and metastasis of cancer (see Non-Patent Document 1), that a pseudo kinase of PTK7 or a fragment thereof in cancer cells is involved in the activation of signal transduction related to cancer, such as Akt or c-Jun signal transduction (see Non Patent Document 2), that PTK7 is involved in the control of a β-catenin-independent pathway of Wnt signal transduction and is involved in a disease related to the up regulation of Wnt signal transduction, and that the up regulation of PTK7 occurs in many cancers (see Non Patent Document 3).

Therefore, PTK7 is being studied as a cancer marker and a medical treatment for cancer. For example, the use of PTK7 as a marker for human colon stem cells by an antibody that recognizes an extracellular domain of PTK7 (see Non Patent Document 4), a therapeutic method through an antibody-drug complex using a PTK7 antibody, which targets tumor progenitor cells of triple-negative breast cancer, ovarian cancer, and lung cancer (see Non Patent Document 5), and a therapeutic method through the activation of anti-cancer immunity obtained by stimulating dendritic cells with an antibody-drug complex of a PTK7 antibody and a microtubule depolymerizing agent (see Non Patent Document 6) and the like are known.

### Citation List

### Non Patent Documents

Non-Patent Document 1: Golubkov V. S. et al., Protein-tyrosine Pseudokinase 7 (PTK7), Directs Cancer Cell Motility and Metastasis., J Biol Chem, 289(35), 24238-24249, 2014.
Non-Patent Document 2: Golubkov V. S. and Strongin A. Y., Downstream Signaling and Genome-Wide Regulatory Effects of PTK7 Pseudokinase and Its Proteolytic Fragments in Cancer Cells., Cell Commun Signal, 112, 15, 2014.
Non-Patent Document 3: Berger H., et al., PTK7 Faces the Wnt in Development and Disease, Front Cell Dev Biol, 5, 31, 2017.
Non-Patent Document 4: Jung P., et al., Isolation of Human Colon Stem Cells Using Surface Expression of PTK7., Stem Cell Reports, 5 (6), 979-987, 2015.
Non-Patent Document 5: Damelin M., et al., A PTK7-targeted antibody-drug conjugate reduces tumor-initiating cells and induces sustained tumor regressions., Sci Transl Med. 9, eaag2611, 2017.
Non-Patent Document 6: Muller P., et al., Microtubule-depolymerizing agents used in antibody-drug conjugates induce antitumor immunity by stimulation of dendritic cells., Cancer Immunol. Res., 2, 741-755, 2014.

### SUMMARY OF INVENTION

### Technical Problem

An object of the present invention is to provide an antibody against PTK7, or a fragment thereof.

### Solution to Problem

The present invention includes the following embodiments.
[1] An antibody or a fragment thereof, in which complementarity determining regions (CDR-H1 to CDR-H3) of a heavy chain variable region (V_{H} region) include each of amino acid sequences set forth in SEQ ID NOs: 1 to 3, and complementarity determining regions (CDR-L1 to CDR-L3) of a light chain variable region (V_{L} region) include each of amino acid sequences set forth in SEQ ID NOs: 4 to 6.
[2] The antibody or the fragment thereof according to [1], in which CDR-H1 to CDR-H3 of the V_{II} region consist of each of the amino acid sequences set forth in SEQ ID NOs: 1 to 3, and
   CDR-L1 to CDR-L3 of the V_{L} region consist of each of the amino acid sequences set forth in SEQ ID NOs: 4 to 6 in no particular order.
[3] The antibody or the fragment thereof according to [1] or [2], in which the V_{H} region includes an amino acid sequence set forth in SEQ ID NO: 7, and CDR-H1 to CDR-H3 in the V_{II} region have each of an amino acid sequence from a position 50 to a position 54, an amino acid sequence from a position 69 to a position 85, and an amino acid sequence from a position 118 to a position 122 in the amino acid sequence set forth in SEQ ID NO: 7, and
   the V_{L} region includes an amino acid sequence set forth in SEQ ID NO: 8, and CDR-L1 to CDR-L3 in the V_{L} region have each of an amino acid sequence from a position 49 to a position 59, an amino acid sequence from a position 75 to a position 81, and an amino acid sequence from a position 114 to a position 122 in the amino acid sequence set forth in SEQ ID NO: 8.
[4] The antibody or the fragment thereof according to any one of [1] to [3], in which the V_{H} region consists of the amino acid sequence set forth in SEQ ID NO: 7, and
   the V_{L} region consists of the amino acid sequence set forth in SEQ ID NO: 8.
[5] The antibody or the fragment thereof according to any one of [1] to [4], in which a framework region (FR_{H}) in the V_{H} region has at least 80% sequence identity to FR_{H} in the continuous amino acid sequence set forth in SEQ ID NO: 7, and
   a framework region (FR_{L}) in the V_{L} region has at least 80% sequence identity to FR_{L} in the continuous amino acid sequence set forth in SEQ ID NO: 8.
[6] The antibody or the fragment thereof according to any one of [1] to [5], in which the antibody or the fragment thereof binds to human PTK7.
[7] An antibody or a fragment thereof, which binds to an epitope including an amino acid sequence from a position 310 to a position 331 and an amino acid sequence from a position 104 to a position 113 in an amino acid sequence set forth in any one of SEQ ID NOs: 9 to 14.
[8] The antibody or the fragment thereof according to [7], in which the epitope consists of an amino acid sequence from a position 310 to a position 331 in the amino acid sequence set forth in any one of SEQ ID NOs: 9 to 14.
[9] The antibody or the fragment thereof according to [7], in which the epitope consists of an amino acid sequence from a position 104 to a position 113 in the amino acid sequence set forth in any one of SEQ ID NOs: 9 to 14.
[10] The antibody or the fragment thereof according to [7], in which the antibody or the fragment thereof binds to an epitope including an amino acid sequence from a position 310 to a position 331 and an amino acid sequence from a position 104 to a position 113 in an amino acid sequence set forth in SEQ ID NO: 11.
[11] The antibody or the fragment thereof according to [7], in which the epitope consists of an amino acid sequence from a position 310 to a position 331 in an amino acid sequence set forth in SEQ ID NO: 11.
[12] The antibody or the fragment thereof according to [7], in which the epitope consists of an amino acid sequence from a position 104 to a position 113 in an amino acid sequence set forth in SEQ ID NO: 11.
[13] An antibody or a fragment thereof, which competes with the antibody or the fragment thereof according to any one of [1] to [12], when being bound to human PTK7.
[14] The antibody or the fragment thereof according to [13], in which the human PTK7 is a protein consisting of an amino acid sequence set forth in any one of SEQ ID NOs: 9 to 14.
[15] The antibody or the fragment thereof according to [14], in which the human PTK7 is a protein consisting of an amino acid sequence set forth in SEQ ID NO: 11.
[16] The antibody or the fragment thereof according to any one of [1] to [15],
   wherein the antibody or the fragment thereof is a chimeric antibody, a humanized antibody, or a human antibody.
[17] An anti-cancer agent containing, as an active ingredient:
   the antibody or the fragment thereof according to any one of [1] to [16].
[18] An anti-cancer pharmaceutical composition containing:
   the anti-cancer agent according to [17]; and
   a pharmaceutically acceptable carrier.
[19] A medical treatment method for cancer, including:
   administering an effective amount of the anti-cancer agent according to [17] or the anti-cancer pharmaceutical composition according to [18] to a patient in need thereof. Advantageous Effects of Invention

According to the present embodiment, it is possible to provide an antibody against human PTK7 or a fragment thereof.

### BRIEF DESCRIPTION OF DRAWINGS

[FIG. 1] FIG. 1 is a graph showing the results obtained by evaluating the of reactivity of a #19 antibody in Experimental Example 3.
[FIG. 2] FIG. 2 is a graph showing the results obtained by evaluating the stability of the #19 antibody in Experimental Example 4.
[FIG. 3] FIG. 3 is a graph showing the results of flow cytometry in Experimental Example 6, in which the expression of PTK7 in HCT116 cells has been examined.
[FIG. 4] FIG. 4 shows a fluorescence photomicrograph showing representative results obtained by examining the reactivity of the #19 antibody with respect to HCT116 cells by immunofluorescence cell staining in Experimental Example 7.
[FIG. 5] FIG. 5 is a graph showing the results obtained by evaluating the reactivity of a #19 chimeric antibody in Experimental Example 10.
[FIG. 6] FIG. 6 is a graph showing the results of flow cytometry in Experimental Example 11, in which the expression of PTK7 in hematological cancer cells has been examined.
[FIG. 7] FIG. 7 is a graph showing the results of flow cytometry in Experimental Example 12, in which the expression of PTK7 in a SCID mouse transplanted with MOLT-4 cells has been examined.
[FIG. 8] FIG. 8 is a graph showing the results obtained by measuring a change over time in a tumor volume of a humanized mouse transplanted with HCT116 cells in Experimental Example 13.
[FIG. 9] FIG. 9 is a graph showing the results obtained by measuring the tumor volume of the humanized mouse transplanted with HCT116 cells in Experimental Example 13.
[FIG. 10] FIG. 10 is a graph showing the results obtained by observing a survival rate of a NOG mouse transplanted with hematopoietic stem cells over time in Experimental Example 13.

### DESCRIPTION OF EMBODIMENTS

In the present specification, the term "antibody" refers to a protein that interacts with an antigen, which includes at least two heavy chains and two light chains, which are linked to each other by a disulfide bond. The heavy chain consists of a heavy chain variable region (V_{II} region) and a heavy chain constant region (C_{II} region), and the light chain consists of a light chain variable region (V_{L} region) and a light chain constant region (C_{L} region). The V_{H} region and the V_{L} region consist of a framework region (FR region) and a complementarity determining region (CDR) having high-frequency variability in a region that comes into direct contact with an antibody. Each of the V_{H} region and the V_{L} region consists of three CDRs and four FRs, which are arranged in the order of FR1, CDR1, FR2, CDR2, FR3, CDR3, and FR4 from the amino terminal to the carboxy terminal. CDR-H1 to CDR-H3 indicate each of CDR1 to CDR3 in the V_{H} region, and CDR-L1 to CDR-L3 indicate each of CDR1 to CDR3 in the V_{L} region. FR_{H} and FR_{L} indicate each of an FR region in the V_{II} region and an FR region in the V_{L} region, FR-H1 to FR-H4 indicate each of FR1 to FR4 of FR_{H}, and FR-L1 to FR-L4 indicate each of FR1 to FR4 of FR_{L}.

In the present specification, an amino acid position assigned to a complementarity determining region of an antibody is defined according to the definition of Kabat (Kabat E., Wu T., Perry H., Gottesman K., Sequences of Proteins of Immunological Interest, National Institute of Health, Bethesda, Md., 1987 and 1991).

In the present specification, the antibody includes isotypes such as IgG, IgE, IgM, IgD, IgA, and IgY, classes such as IgG1, IgG2, IgG3, IgG4, IgA1, and IgA2, and subclasses thereof.

In the present specification, the term "antibody fragment" indicates one or more portions included in the antibody, where the antibody fragment has an ability to bind to human PTK7. Examples of the antibody fragment include F(ab')₂ (a fragment including two Fab fragments linked by disulfide crosslinking in a hinge region), Fab (a fragment consisting of V_{L}, V_{H}, C_{L}, and C_{H}1), Fv (a fragment consisting of V_{L} and V_{H} domains of a single arm of an antibody), Fd (a fragment consisting of V_{H} and C_{H}1 domains), CDR-H1 to H3, and CDR-L1 to L3. The antibody fragment also includes a mixture of one kind or a plurality of kinds thereof.

In the present specification, an amino acid sequence indicated by a sequence identification number includes an amino acid sequence subjected to a conservative substitution. The term "conservative substitution" refers to substituting any amino acid residue with another amino acid residue having properties that are chemically and/or biologically similar to the properties of the residue to be substituted. Examples of the substitution include a substitution between acidic residues, a substitution between basic residues, a substitution between hydrophilic non-charged residues, a substitution between aliphatic non-charged residues, a substitution between non-polar and non-charged residues, and a substitution between aromatic residues. Examples of the substitution include substitution within the following combinations: Val, Ile, Leu, and Met; Asp and Glu; Gly and Ala; Ser and Thr; Asn and Gln; Lys and Arg; Cys and Pro; and Phe and Tyr.

### [Antibody or fragment thereof]

In one embodiment, the present invention provides an antibody or a fragment thereof, in which complementarity determining regions (CDR-H1 to CDR-H3) of a heavy chain variable region (V_{II} region) include each of amino acid sequences set forth in SEQ ID NOs: 1 to 3, and complementarity determining regions (CDR-L1 to CDR-L3) of a light chain variable region (V_{L} region) include each of amino acid sequences set forth in SEQ ID NOs: 4 to 6. Hereinafter, the present embodiment will also be referred to as "embodiment 1".

As described later in Examples, the antibody or the fragment thereof according to the present embodiment can bind to a human protein tyrosine kinase 7 (human PTK7) expressed in a cancer cell.

The GeanBank accession number and amino acid sequence of the human PTK7 protein are EAX04154.1 (SEQ ID NO: 9), EAX04155.1 (SEQ ID NO: 10), EAX04156.1 (SEQ ID NO: 11), EAX04158.1 (SEQ ID NO: 12), EAX04159.1 (SEQ ID NO: 13), and EAX04160.1 (SEQ ID NO: 14).

In the present specification, the amino acid sequence set forth in any of SEQ ID NOs: 9 to 14 includes an amino acid sequence which has been subjected to a deletion or addition, in addition to the amino acid sequence subjected to the conservative substitution, where the amino acid sequence is an amino acid sequence having the same function as the protein consisting of the amino acid sequence set forth in any of SEQ ID NOs: 9 to 14.

CDR-H1 includes a sequence of five consecutive amino acids set forth in SEQ ID NO: 1, CDR-H2 includes a sequence of seventeen consecutive amino acid set forth in SEQ ID NO: 2, CDR-H3 includes a sequence of five consecutive amino acid set forth in SEQ ID NO: 3, CDR-L1 includes a sequence of eleven consecutive amino acid set forth in SEQ ID NO: 4, CDR-L2 includes a sequence of seven consecutive amino acid set forth in SEQ ID NO: 5, and CDR-L3 includes a sequence of nine consecutive amino acid set forth in SEQ ID NO: 6.

Preferably, CDR-H1 consists of five consecutive amino acid sequences set forth in SEQ ID NO: 1, CDR-H2 consists of seventeen consecutive amino acid sequences set forth in SEQ ID NO: 2, CDR-H3 consists of five consecutive amino acid sequences set forth in SEQ ID NO: 3, CDR-L1 consists of eleven consecutive amino acid sequences set forth in SEQ ID NO: 4, CDR-L2 consists of seven consecutive amino acid sequences set forth in SEQ ID NO: 5, and CDR-L3 consists of nine consecutive amino acid sequences set forth in SEQ ID NO: 6. The amino acid sequences of SEQ ID NOs: 1 to 6 are derived from a rat-origin sequence.

In one embodiment, the present invention provides an antibody or a fragment thereof, in which the V_{II} region contains an amino acid sequence set forth in SEQ ID NO: 7, where the amino acid sequence of the V_{H} region preferably consists of the amino acid sequence set forth in SEQ ID NO:7, CDR-H1 to CDR-H3 in the V_{H} region have each of an amino acid sequence from a position 50 to a position 54, an amino acid sequence from a position 69 to a position 85, and an amino acid sequence from a position 118 to a position 122 in SEQ ID NO: 7, the V_{L} region contains an amino acid sequence set forth in SEQ ID NO: 8, where the V_{L} region preferably consists of the amino acid sequence set forth in SEQ ID NO: 8, and CDR-L1 to CDR-L3 in the V_{L} region have each of an amino acid sequence from a position 49 to a position 59, an amino acid sequence from a position 75 to a position 81, and an amino acid sequence from a position 114 to a position 122 in the amino acid sequence set forth in SEQ ID NO: 8. Hereinafter, the present embodiment will also be referred to as "embodiment 2".

In a case where the V_{II} region consists of the amino acid sequence set forth in SEQ ID NO: 7, FR-H1 to FR-H4 have each of amino acid sequences from a position 1 to a position 49, from a position 55 to a position 68, from a position 86 to a position 117, and from a position 123 to a position 133 in the amino acid sequence set forth in SEQ ID NO: 7.

In a case where the V_{II} region consists of the amino acid sequence set forth in SEQ ID NO: 7, FR-H1 to FR-H4 have each of amino acid sequences from a position 1 to a position 48, from a position 60 to a position 74, from a position 82 to a position 113, and from a position 123 to a position 133 in the amino acid sequence set forth in SEQ ID NO: 7.

The antibody of the embodiment 2 includes an antibody in which FR_{II} has at least 80%, 85%, 90%, or 95% sequence identity to FR_{H} in the continuous amino acid sequence set forth in SEQ ID NO: 7, and at least, FR_{L} has at least 80%, 85%, 90%, or 95% sequence identity to FR_{L} in the continuous amino acid sequence set forth in SEQ ID NO: 8.

In the present specification, the sequence identity means a percentage of the number of matching nucleotides or amino acids shared between two sequences in a case where two sequences of amino acid sequences are aligned in an optimal aspect.

In one embodiment, the present invention provides an antibody or a fragment thereof, which binds to an epitope including an amino acid sequence from a position 310 to a position 331 and an amino acid sequence from a position 104 to a position 113 in an amino acid sequence set forth in any one of SEQ ID NOs: 9 to 14. In this case, the epitope of human PTK7 is a discontinuous epitope. Hereinafter, the present embodiment will also be referred to as "embodiment 3".

The amino acid sequences of SEQ ID NOs: 9 to 14 are an amino acid sequence of a representative variant of human PTK7. The epitope means an amino acid residue that is capable of directly and specifically binding to an antibody. In addition, the epitope usually has a three-dimensional structural feature and an electrical charge feature.

The epitope can be specified by appropriately combining publicly known methods such as an epitope mapping method, a Western blotting method, and an immunoprecipitation method. **In** addition, the spatial conformation of the epitope can be specified by appropriately combining publicly known methods such as an X-ray crystal structure analysis method and a 2D nuclear magnetic resonance method.

The epitope may be an epitope that consists of an amino acid sequence from a position 310 to a position 331 in the amino acid sequence set forth in any one of SEQ ID NOs: 9 to 14. Alternatively, the epitope may be an epitope that consists of an amino acid sequence from a position 104 to a position 113 in the amino acid sequence set forth in any one of SEQ ID NOs: 9 to 14.

The amino acid sequence of human PTK7 is preferably the amino acid sequence set forth in SEQ ID NO: 11.

In one embodiment, the present invention provides an antibody or a fragment thereof, which competes with the antibodies or fragments thereof according to the embodiments 1 to 3, when being bound to human PTK7.

Hereinafter, the present embodiment will also be referred to as "embodiment 4".

The term "compete" indicates that in the presence of another antibody or a fragment thereof, a certain antibody or a fragment thereof has a property of being equally or more likely to bind to a certain binding partner compared to the other antibody or a fragment thereof. It is possible to evaluate whether a certain antibody or a fragment thereof is the antibody or a fragment thereof according to the embodiment 4, by carrying out the evaluation with an ELISA analysis or a FACS analysis by using the certain antibody or a fragment thereof, the antibodies or fragments thereof according to the embodiments 1 to 3, and the human PTK7 antibody.

The antibodies according to the embodiments 1 to 4 include an antibody derived from a non-human animal, a chimeric antibody, a humanized antibody, and a human antibody. The chimeric antibody has CDR-H1 to CDR-H3, CDR-L1 to CDR-L3, FR-H1 to FR-H4, and FR-L1 to FR-L4, which are derived from sequences of non-human animal origin, and a C_{H} region and a C_{L} region, which are derived from sequences of human origin. The humanized antibody has CDR-H1 to CDR-H3 and CDR-L1 to CDR-L3, which are derived from sequences of non-human animal origin, and FR-H1 to FR-H4 and FR-L1 to FR-L4, which are derived from sequences of human origin, as well as a C_{II} region and a C_{L} region. The human antibody has CDR-L1 to CDR-L3, FR-H1 to FR-H4, and FR-L1 to FR-L4, which are derived from sequences of human origin, as well as a C_{H} region and a C_{L} region.

The dissociation constants (K_{D}) of the antibodies or fragments thereof according to the embodiments 1 to 4 are generally 10⁻² M or less, 10⁻³ M or less, 10⁻⁴ M or less, 10⁻⁵ M or less, 10⁻⁶ M or less, 10⁻⁷ M or less, 10⁻⁸ M or less, 10⁻⁹ M or less, 10⁻¹⁰ M or less, or 10⁻¹¹ M or less. The dissociation constant can be measured by surface plasmon resonance or isothermal titration type calorimetry.

The antibodies according to the embodiments 1 to 4 are preferably an isolated antibody. The "isolated antibody" indicates an antibody that does not substantially include other antibodies having different antigen specificity, and it is preferably a monoclonal antibody.

An antibody derived from a non-human animal can be produced by a hybridoma method. That is, a cell (hybridoma) obtained by fusing a non-human animal-derived B cell producing a monoclonal antibody with a cancer cell such as a myeloma cell is prepared, and the antibody activity of the culture supernatant thereof is examined, whereby a desired clone can be acquired.

The chimeric antibody is produced by analyzing a gene sequence of an antibody of a mouse, a rat, or the like, which is obtained by a hybridoma method, and replacing at least a part of a constant region with a gene sequence of a human antibody by gene recombination.

The humanized antibody is produced by analyzing a gene sequence of a monoclonal antibody of a mouse, a rat, or the like, which is obtained by a hybridoma method, and replacing a constant region and a framework region with a gene sequence of a human antibody by gene recombination.

The human antibody means an antibody, the entirety of which is derived from a human including the CDR. The human antibody can be produced by a method of carrying out screening from a human antibody gene library by a display method such as a phage display method, a ribosome display method, or a yeast display method, a method of producing a human antibody by immunizing a genetically modified mouse holding a human Ig gene locus, or the like.

In a case where the antibody or the fragment thereof according to the present embodiment is a chimeric antibody or a fragment thereof, a humanized antibody or a fragment thereof, or a human antibody or a fragment thereof, immunogenicity in a case of administration to a human can be reduced, and side effects such as anaphylactic shock can be suppressed, and as a result, administration to a human is possible.

### [Anti-cancer agent and anti-cancer pharmaceutical composition]

In one embodiment, the present invention provides an anti-cancer agent containing the above-mentioned antibody or a fragment thereof as an active ingredient. As described later in Examples, cancer can be treated by administering the anti-cancer agent according to the present embodiment.

Examples of the cancer to be treated by the anti-cancer agent according to the present embodiment include a cancer in which the expression of PTK7 is up-regulated. Examples of the cancer in which the expression of PTK7 is up-regulated include colon cancer, rectal cancer, acute lymphoblastic leukemia, T-cell leukemia, lung cancer, breast cancer, esophageal cancer, thyroid cancer, gastric cancer, oral cancer, cervical cancer, intrahepatic bile duct cancer, prostate cancer, and liposarcoma.

The anti-cancer agent according to the present embodiment includes the anti-PTK7 antibody according to the present embodiment and an antibody-drug complex in which the anti-PTK7 antibody is complexed with a drug having pharmacological activity against cancer.

It is preferable that the anti-cancer agent according to the present embodiment is mixed with a pharmaceutically acceptable carrier to be formulated as an anti-cancer pharmaceutical composition. Examples of the pharmaceutically acceptable carrier include a buffer solution such as a neutral buffered saline or a phosphate buffered saline; a carbohydrate such as glucose, mannose, sucrose, dextran, or mannitol; and an amino acid such as glycine; however, the pharmaceutically acceptable carrier is not limited thereto.

The anti-cancer pharmaceutical composition may contain additives such as an antioxidant, a chelating agent, and a preservative. The anti-cancer pharmaceutical composition is preferably formulated for intravenous administration.

### [Other embodiments]

In one embodiment, the present invention provides a method for treating cancer, which includes administering an effective amount of the above-described anti-cancer agent or the above-described anti-cancer pharmaceutical composition to a patient in need thereof.

The administration dose of the above-described anti-cancer agent or the above-described anti-cancer pharmaceutical composition varies depending on the symptoms, weight, age, sex, and the like of the patient, and thus it cannot be determined unconditionally; however, it is conceivable to administer 0.001 to 500 mg of the active ingredient (antibody or fragment thereof) per administration by intravenous drip infusion at intervals of 1 to 5 weeks.

In one embodiment, the present invention provides the above-described antibody or fragment thereof for the medical treatment for cancer.

In one embodiment, the present invention provides use of the above-described antibody or a fragment thereof for producing an anti-cancer agent.

### Examples

Hereinafter, the present embodiment will be described in more detail based on Examples. However, the present embodiment is not limited to these Examples.

### [Experimental Example 1]

### (Production of human PTK7-expressing HEK293T cells)

A human PTK7 gene was introduced into HEK293T cells, which are a human fetal kidney cell line, and human PTIK7-expressing HEK293 cells were produced. HEK293T cells (cell line name "ATCC^{(R)} CRL-3216") were seeded in an expansion culture medium and expanded at 37°C for 1 day (60% to 70% confluency). A culture medium containing DMEM, fetal bovine serum (FBS), and antibiotics was used as the expansion culture medium.

Subsequently, a human PTK7 (full length) expression plasmid (pcDNA3-PTK7-VSV) was introduced into the HEK293T cells. A liquid (a) consisting of OptiMEM^{(R)} (Thermo Fisher Scientific, Inc., 250 µL) and pcDNA3-PTK7-VSV (5 to 7 µg) and a liquid (b) consisting of OptiMEM^{(R)} (Thermo Fisher Scientific, Inc., 250 µL) and polyethyleneimine (PEI, 25 to 35 µL) were prepared, and each of the liquids was pipetted and then allowed to stand at room temperature (23°C) for 5 minutes.

The (a) liquid and the (b) liquid were mixed and allowed to stand at room temperature for 15 minutes. The mixed liquid was added to the culture medium of the HEK293T cells which had been subjected to expansion culture, and the cells were cultured overnight at 37°C. The expansion culture medium was exchanged, and the cells were further cultured at 37°C for 1 day. The HEK293T cells after culturing were recovered and washed twice with phosphate buffered saline (PBS) to obtain human PTK7-expressing HEK293T cells.

### [Experimental Example 2]

### (Production of hybridoma and acquisition of #19 antibody)

A rat (Wister rat) was immunized three times with the human PTK7-expressing HEK293T cells produced in Experimental Example 1, and B cells were obtained. The B cells and myeloma cells were fused, and a hybridoma was produced by a conventional method.

A #19 antibody was selected as a monoclonal antibody having reactivity based on the examination results from an ELISA method using a fragment of human PTK7 consisting of amino acid residues from a position 31 to a position 702 of the genetically modified human PTK7 (SEQ ID NO: 11) and reactive flow cytometry of the human PTK7-expressing HEK293T cells, and the examination results of reactivity of magnetic particles to the human PTK7-expressing HEK293T cells, where the monoclonal antibody in the culture supernatant of the hybridoma was bound to the magnetic particles.

The hybridoma was activated with a culture solution for waking up (Hybridoma-SFM, 10% FBS, and 1% penicillin-streptomycin) and put into a serum-free culture medium (Hybridoma-SFM and 1% penicillin-streptomycin) for serum-free adaptation. The adapted cells were seeded in a roller bottle and cultured until the viable cell rate reached 10% or less. The culture supernatant was collected and filtered through a 0.22 µm filter to purify the monoclonal antibody (#19 antibody) with a protein G column. The purity was checked by CBB staining after SDS-PAGE.

A genetically modified human PTK7, which was obtained by subjecting Expi293T cells (Thermo Fisher Scientific, Inc.) to gene introduction with a plasmid (pSec-Tag2-PTK7 (31-702)) by using PEI, and purifying recombinant PTK7 (31-702) secreted into the culture supernatant, was used.

### [Experimental Example 3]

### (Checking of binding activity of #19 antibody to human PTK7 by ELISA method)

The binding activity of the #19 antibody to human PTK7 was evaluated by the ELISA method. The following materials were used.
96-well plate: product name "MaxiSorp surface treatment Nunc-Immuno Module, F8", Cosmo Bio Company, Limited.
Buffer solution 1 (TBS-T): Tween 20-containing Tris-buffered saline.
Buffer solution 2: A buffer solution prepared by diluting product name "Blocking One" with TBS-T such that the concentration thereof was 5 w/v%.
Blocking buffer solution: A buffer solution prepared by diluting "Blocking One" (product name, manufactured by Nacalai Tesque, Inc.) with TBS-T such that the concentration thereof was 20 w/v%.
Antigen diluted solution: An antigen diluted solution obtained by diluting the fragment of human PTK7 of Experimental Example 2 with Tris-buffered saline (TBS) such that the concentration thereof was 1 µg/mL.
Primary antibody diluted solution: A primary antibody diluted solution obtained by diluting the #19 antibody with the buffer solution 2 such that the concentration thereof was 0.001 to 1 µg/mL.
Secondary antibody diluted solution: A secondary antibody diluted solution obtained by diluting Donkey Anti-Rat IgG H&L (HRP) (Abcam plc) with the buffer solution 2.
ELISA substrate solution: product name "1-Step^{™} Ultra TMB-ELISA Substrate" (Thermo Fisher Scientific, Inc.).

An antigen diluted solution (50 µL/well) was added to the wells of a 96-well plate, and the plate was allowed to stand at 4°C overnight. After washing the wells 5 times with TBS-T, a blocking buffer solution (200 µL/well) was added to the wells, and the wells were allowed to stand at 4°C for more than 2 hours. After washing the wells 5 times with TBS-T, a primary antibody diluted solution (50 µL/well) was added to the wells, and shaking was carried out at room temperature for 1 hour. The wells were washed 5 times with TBS-T, a secondary antibody diluted solution (50 µL/well) was added thereto, and shaking was carried out at room temperature for 1 hour. The wells were washed 5 times with TBS-T, an ELISA substrate solution (50 µL/well) was added thereto, and the wells were allowed to stand at room temperature for 5 minutes. A 2 N HCl aqueous solution (50 µL/well) was added thereto, and the absorbance at a wavelength of 450 nm was measured.

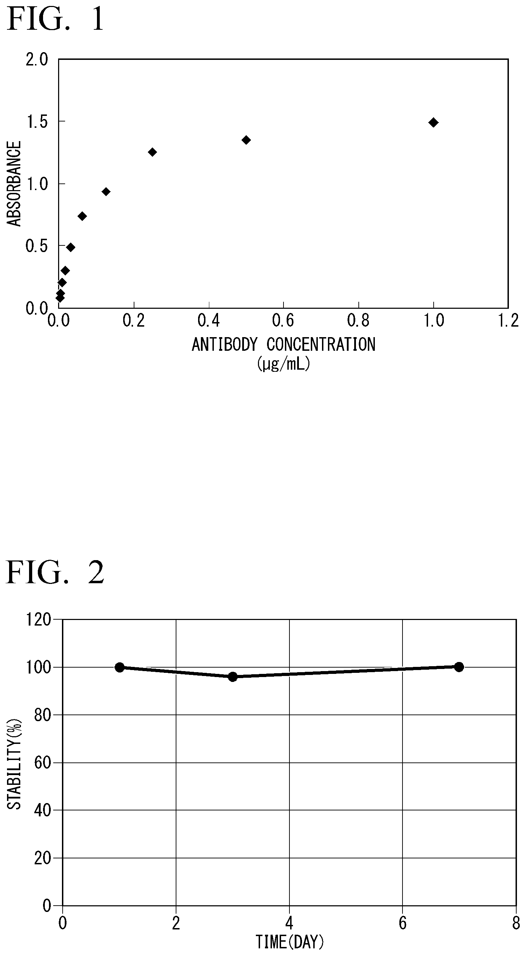

FIG. 1 is a graph showing the results of measuring the absorbance. The vertical axis indicates the absorbance, and the horizontal axis indicates the antibody concentration (µg/mL). As a result, it was revealed that the #19 antibody recognizes the fragment of human PTK7 in a concentration-dependent manner.

### [Experimental Example 4]

### (Evaluation of stability of #19 antibody)

The #19 antibody was stored at 37°C for 1 to 7 days, and the stability was evaluated by the ELISA method. The stability was evaluated by the antigen reactivity (absorbance) of the #19 antibody stored at 37°C with respect to the antigen reactivity (absorbance) of the #19 antibody stored at 4°C.

The #19 antibody was diluted with mouse serum (product name "Normal Mouse Serum", manufactured by FUJIFILM Wako Pure Chemical Corporation) so that the concentration thereof was 1 mg/mL. An antigen diluted solution (50 µL/well) was added to the wells of a 96-well plate, and the plate was allowed to stand at 4°C overnight. After washing the wells 5 times with TBS-T, a blocking buffer solution (200 µL/well) was added to the wells, and the wells were allowed to stand at 4°C for more than 2 hours. After washing the wells 5 times with TBS-T, 50 µL/well of the #19 antibody stored at 37°C or the #19 antibody stored at 4°C was added to the wells, and shaking was carried out at room temperature for 1 hour. The wells were washed 5 times with TBS-T, a secondary antibody diluted solution (50 µL/well) was added thereto, and shaking was carried out at room temperature for 1 hour. The wells were washed 5 times with TBS-T, an ELISA substrate solution (50 µL/well) was added thereto, and the wells were allowed to stand at room temperature for 5 minutes. A 2 N HCl aqueous solution (50 µL/well) was added thereto, and the absorbance at a wavelength of 450 nm was measured.

FIG. 2 is a graph showing the evaluation results of the stability. The vertical axis indicates stability (%) indicates (absorbance of #19 antibody stored at 37°C/absorbance of #19 antibody stored at 4°C × 100), and the horizontal axis indicates storage time (days).

As a result, it was revealed that the #19 antibody is stable.

### [Experimental Example 5]

### (Analysis of CDR of #19 antibody)

Total RNA was extracted from the hybridoma (#19) selected in Experimental Example 2, cDNA was synthesized using a GeneRacer kit (Thermo Fisher Scientific, Inc.), and gene fragments of a rat IgG antibody heavy chain gene and a rat κ chain light chain gene were subjected to PCR amplification.

For the heavy chain, a part of the variable region and a part of the constant region were recovered, and for the light chain, the full length of the variable region and the full length of the constant region were recovered. CDR was predicted based on the Kabat rule.

The amino acid sequence of the #19 antibody heavy chain variable region is set forth in SEQ ID NO: 7, and the amino acid sequence of the #19 antibody light chain variable region is set forth in SEQ ID NO: 8. In addition, the amino acid sequences of the heavy chain CDRs 1 to 3 of the #19 antibody are set forth in each of SEQ ID NOs: 1 to 3, and the amino acid sequences of the light chain CDRs 1 to 3 of the #19 antibody are set forth in each of SEQ ID NOs: 4 to 6.

### [Experimental Example 6]

### (Checking of expression of PTK7 in HCT116 cells by flow cytometry)

The expression of PTK7 in HCT116 cells, which are a human colon adenocarcinoma cell line, was checked. The #19 antibody (concentration: 10 µg/mL, diluted with 100 µL of PBS) was added to a cell suspension containing 1 × 10⁶ HCT116 cells, and the cells were allowed to stand on ice for 15 minutes. In addition, as a control, the same operation was carried out using an isotype control antibody. After washing the HCT116 cells with PBS, an Alexa Fluor 488-labeled secondary antibody (product name, "anti-rat IgG Alexa Fluor 488", Thermo Fisher Scientific, Inc.) was added thereto, and the cells were allowed to stand on ice for 15 minutes. After washing the HCT116 cells with PBS, the HCT116 cells were resuspended and filtered through a filter (BD Biosciences) having a pore size of 40 µm. Subsequently, the fluorescence of Alexa Fluor 488 was measured using a flow cytometer (product name, "BD LSR Fortessa", BD Biosciences).

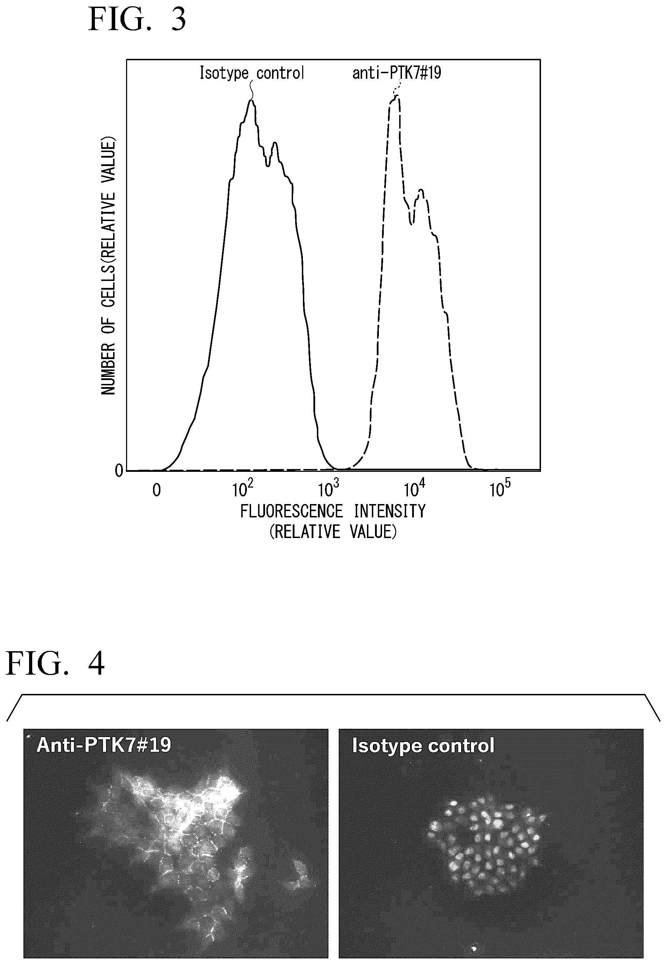

FIG. 3 is a graph showing the results of flow cytometry. The vertical axis indicates the number of cells (relative value), and the horizontal axis indicates the fluorescence intensity (relative value). In FIG. 3, "anti-PTK7#19" indicates the result of the #19 antibody, and "Isotype control" indicates the result of the isotype control antibody. As a result, it was confirmed that the HCT116 cells express PTK7.

### [Experimental Example 7]

### (Examination of reactivity of #19 antibody with respect to HCT116 cell)

The reactivity of the #19 antibody was examined by immunofluorescence cell staining. A cover glass was placed in a well of a 12-well plate, and 1 × 10⁵ HCT116 cells were seeded on the cover glass. Subsequently, the cells were cultured in an incubator at 37°C and 5 v/v% CO₂ for 24 hours.

A primary antibody diluted solution was added to the well, and the cells were allowed to stand overnight in an incubator at 37°C and 5 v/v% CO₂. As the primary antibody diluted solution, a solution obtained by diluting the #19 antibody with PBS to 20 µg/mL was used. In addition, as a control, a rat IgG2a isotype control antibody diluted to 20 µg/mL with PBS was used.

The HCT116 cells were washed three times with PBS, 4 w/v% paraformaldehyde was added thereto, and the cells were allowed to stand in an incubator at 37°C for 10 minutes to be fixed. Subsequently, a blocking solution was added thereto, and the wells were allowed to stand at room temperature for 30 minutes. As the blocking solution, skim milk (MEGMILK SNOW BRAND Co., Ltd.) diluted with PBS to 5 w/v% was used.

The HCT116 cells were washed three times with PBS. Subsequently, a secondary antibody diluted solution was added thereto, and the wells were allowed to stand at room temperature for 30 minutes. As the secondary antibody diluted solution, anti-rat IgG Alexa Fluor 488 (Thermo Fisher Scientific, Inc.) diluted with PBS was used. The HCT116 cells were washed three times with PBS. The nuclei were stained by adding 4',6-diamidino-2-phenylindole (DAPI), and the HCT116 cells were washed three times with PBS.

The cover glass was taken out, and the slide glass was sealed with a sealing agent (product name "VECTASHIELD Mounting Medium", Vector Laboratories, Inc.).

The stained HCT116 cells were observed using a fluorescence microscope (product name "BZ-9000", manufactured by KEYENCE CORPORATION). FIG. 4 shows a fluorescence photomicrograph. The left side of FIG. 4 shows the results obtained using the #19 antibody, and the right side of FIG. 4 shows the results obtained using the isotype control antibody. As a result, it was revealed that the #19 antibody reacts with PTK7 expressed on the cell membrane of the HCT116 cell.

### [Experimental Example 8]

### (Epitope analysis)

The #19 antibody was immobilized on magnetic particles (product name "Magnoshere MS300/Carboxyl", SR Life Sciences, LLC) according to a covalent binding method by amino coupling. The #19 antibody was reacted with an antigen (PTK7, SEQ ID NO: 11) or a synthetic peptide (a peptide consisting of 38 amino acid residues of EIEDMPLFEPRVFTAGSEERVTCLPPKGLPEPSVWWEH (SEQ ID NO: 15)), and then the antigen was degraded using a digestive enzyme. After the degradation, a fragment of PTK7 bound to the #19 antibody was extracted, subjected to a desalting treatment, and then subjected to mass spectrometry using ultra performance liquid chromatography (UPLC, product name "ACOQITY M-CLASS Nano LC", Waters Corporation).

The peaks presumed to be derived from the epitope were identified by analyzing the results of the mass spectrometry by Total Ion Chromatogram. In addition, the results of the mass spectrometry were analyzed by PEAKS (BSI Inc.) to identify the epitope. The validity of the experimental results was analyzed by carrying out a modeling analysis using an integrated computational chemistry system MOE (Molecular Operating Environment, CCG Inc.).

From these results, it was evaluated that the epitope of PTK7 for the antibody #19 includes DVTGEEAR (SEQ ID NO: 16) from a position 331 to a position 310 of any of the amino acid sequences of SEQ ID NOs: 9 to 14 and/or VFTAGSEER (SEQ ID NO: 17) from a position 104 to a position 113 of any of the amino acid sequences of SEQ ID NOs: 9 to 14.

### [Experimental Example 9]

### (Production of chimeric antibody)

The gene fragment of the heavy chain variable region of the #19 antibody, where the heavy chain variable region was identified in Experimental Example 5, was incorporated into a plasmid DNA of a human antibody heavy chain (IgG1) to prepare a chimeric antibody heavy chain plasmid DNA. In addition, the gene fragment of the light chain variable region was incorporated into a plasmid DNA of a human antibody light chain to prepare a chimeric antibody light chain plasmid DNA.

Subsequently, a CHO cell line derived from a Chinese hamster ovary was subjected to gene introduction with the plasmid DNAs of the chimeric antibody heavy chain and the chimeric antibody light chain. The culture supernatant of the CHO cells subjected to the gene introduction was purified, and a chimeric antibody (hereinafter, may be referred to as a "#19 chimeric antibody") was acquired from the culture supernatant.

### [Experimental Example 10]

### (Checking of binding activity of #19 chimeric antibody to human PTK7 by ELISA method)

The binding activity of the #19 chimeric antibody to human PTK7 was examined according to the same operation as in Experimental Example 3, except that a primary antibody diluted solution obtained by diluting the #19 chimeric antibody with the buffer solution 2 such that the concentration thereof was 0.001 to 1 µg/mL and a secondary antibody diluted solution obtained by diluting the product name "Goat anti-human IgG H&L (HRP)" (MEDICAL & BIOLOGICAL LABORATORIES CO., LTD.) with the buffer solution 2 were used. In addition, for comparison, the binding activity of the #19 antibody to human PTK7 was also measured according to the same operation as in Experimental Example 3.

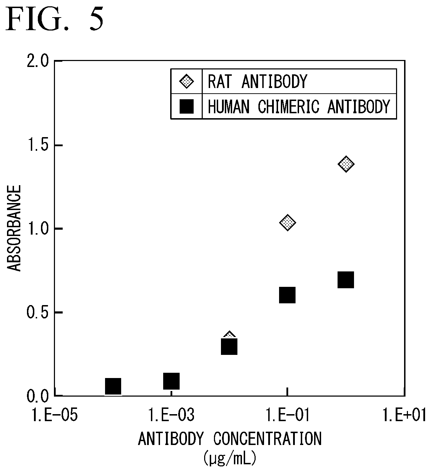

FIG. 5 is a graph showing results obtained by measuring the absorbance. The vertical axis indicates the absorbance, and the horizontal axis indicates the antibody concentration (µg/mL). In FIG. 5, "rat antibody" indicates a #19 antibody, and "human chimeric antibody" indicates a #19 chimeric antibody. As a result, it was revealed that the #19 chimeric antibody exhibits the same binding activity as the #19 antibody with respect to the fragment of human PTK7.

### [Experimental Example 11]

### (Examination of expression of PTK7 in hematological cancer cell)

The expression of PTK7 in hematological cancer cells was examined. MOLT-4, which is a cell line derived from a human acute lymphoblastic leukemia patient, CCRF-CEM, which is a cell line derived from a human acute lymphoblastic leukemia patient, and Jurkat, which is a cell line derived from a human T-cell leukemia patient, were used as hematological cancer cells.

For a specimen containing 1 × 10⁶ hematological cancer cells, the #19 antibody was used as a primary antibody and reacted with a Dylight 488-labeled secondary antibody as a secondary antibody to prepare a Dylight 488-labeled #19 antibody.

In addition, for comparison, a specimen in which a Dylight 488-labeled isotype control antibody was reacted was prepared.

After washing with PBS, the cells were resuspended and filtered through a filter (BD Biosciences) having a pore size of 40 µm. Subsequently, the fluorescence of Dylight 488 was measured using a flow cytometer (product name, "BD LSR Fortessa", BD Biosciences).

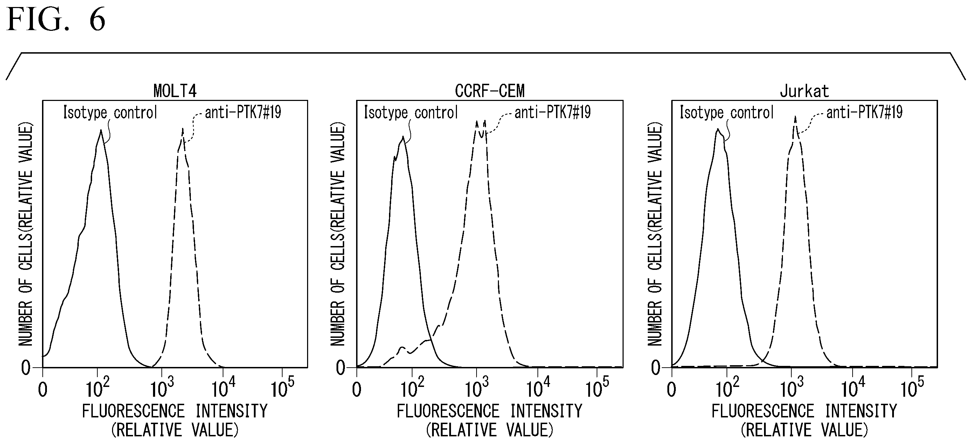

FIG. 6 is a graph showing the results of flow cytometry. The vertical axis indicates the number of cells (relative value), and the horizontal axis indicates the fluorescence intensity (relative value). In FIG. 6, "Isotype control" indicates the result of the isotype control antibody, and "anti-PTK7#19" indicates the result of the #19 antibody. The left side of FIG. 6 shows the results of MOLT-4 cells, the center of FIG. 6 shows the results of CCRF-CEM cells, and the right side of FIG. 6 shows the results of Jurkat cells.

As a result, it was confirmed that the MOLT-4 cells, the CCRF-CEM cells, and the Jurkat cells express PTK7.

### [Experimental Example 12]

### (Checking of expression of PTK7 in SCID mouse transplanted with MOLT-4 cells)

A SCID mouse in which MOLT-4 cells were transplanted into the abdominal cavity was prepared, and the expression of PTK7 in the intraperitoneal cells was checked. First, 1 × 10⁷ MOLT-4 cells were intraperitoneally transplanted into a SCID mouse (severe combined immunodeficiency mouse, female, 8 weeks old), and 28 days after the transplantation, the cells were collected by peritoneal lavage.

A Dylight 488-labeled #19 antibody, a PE-labeled anti-human CD45 antibody (BioLegend, Inc.), and a BV421-labeled anti-mouse CD45 antibody (BD Biosciences) were added to a specimen containing 1 × 10⁶ intraperitoneal cells, and the cells were allowed to stand on ice for 15 minutes.

After washing with PBS, the cells were resuspended and filtered through a filter (BD Biosciences) having a pore size of 40 µm. Subsequently, the fluorescence of each of Dylight 488, PE, and BV421 was measured using a flow cytometer (product name, "BD LSR Fortessa", BD Biosciences).

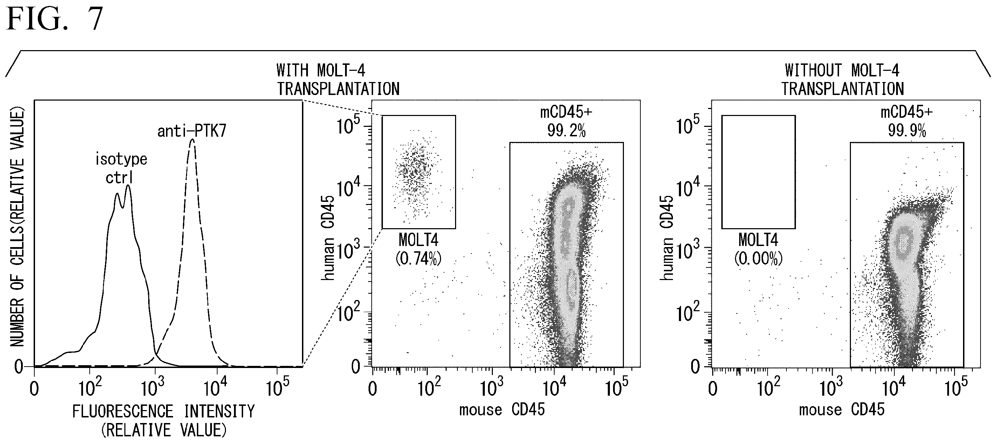

FIG. 7 is a graph showing the results of flow cytometry. The center of FIG. 7 shows a graph showing the expression of human CD45 and mouse CD45 in the intraperitoneal cells derived from the SCID mouse in which MOLT-4 cells were transplanted into the abdominal cavity. As a result, 0.74% of human CD45-positive and mouse CD45-negative cells were detected. These cells are the MOLT-4 cells.

The left of FIG. 7 is a graph showing cells obtained by gating human CD45-positive and mouse CD45-negative cells in the center of FIG. 7, where the Dylight 488-labeled #19 antibody has been bound to the cells. The vertical axis indicates the number of cells (relative value), and the horizontal axis indicates the fluorescence intensity (relative value). In addition, the results obtained by carrying out staining with an isotype control antibody are also shown for comparison.

As a result, it was confirmed that the human CD45-positive and mouse CD45-negative cells in the center of FIG. 7 exhibit reactivity to the Dylight 488-labeled #19 antibody and thus express PTK7.

The right of FIG. 7 shows a graph showing the expression of human CD45 and mouse CD45 in the intraperitoneal cells derived from the SCID mouse to which MOLT-4 cells were transplanted. As a result, it was confirmed that human CD45-positive and mouse CD45-negative cells are not present.

### [Experimental Example 13]

### (Evaluation of anti-cancer activity of #19 antibody in humanized mouse transplanted with HCT116 cells)

The #19 antibody was administered to a humanized mouse transplanted with HCT116 cells, and the anti-cancer activity was evaluated. A NOG mouse transplanted with cord blood-derived CD34-positive cells (a NOG mouse transplanted with hematopoietic stem cells) and a NOG mouse were used as the humanized mice.

Each humanized mouse was anesthetized with isoflurane (Pfizer Inc.), and 1 × 10⁶ HCT116 cells were transplanted into the left and right subcutaneous abdominal parts. Subsequently, the #19 antibody or a rat IgG2a isotype control antibody (5 mg/kg) was intravenously administered to the tail according to a schedule of administration of once per week. Subsequently, the size of the engrafted tumor was measured, and the volume of the tumor was calculated according to Expression (1). Tumor volume = (maximum diameter) × (minimum diameter) × (minimum diameter)/2

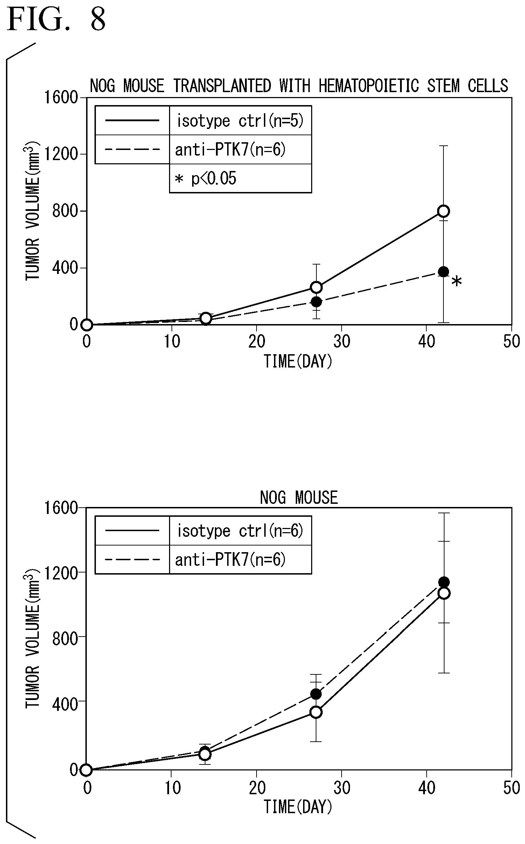

FIG. 8 is a graph showing the results obtained by measuring the change in tumor volume over time. The upper part of FIG. 8 shows the results of the NOG mouse transplanted with hematopoietic stem cells, and the lower part of FIG. 8 shows the results of the NOG mouse. In FIG. 8, "anti-PTK7" indicates the results obtained by administering the #19 antibody. In addition, "isotype ctrl" indicates the results obtained by administering an isotype control antibody. In addition, "*" indicates p < 0.05 and indicates that there is a significant difference.

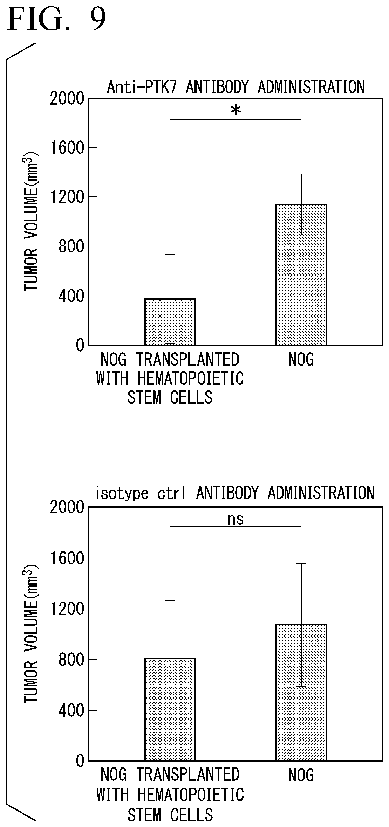

FIG. 9 is a graph showing the tumor volume of each group of mice 42 days after the transplantation of HCT116 cells. The upper part of FIG. 9 shows the results of mice in each group to which the #19 antibody was administered, and the lower part of FIG. 9 shows the results of mice in each group to which the isotype control antibody was administered. In the upper part of FIG. 9, "*" is for p < 0.05 and indicates that there is a significant difference.

As a result, a significant suppression of an increase in tumor volume was observed in a case where the #19 antibody was administered to the NOG mouse transplanted with hematopoietic stem cells. This result indicates that the #19 antibody has an anti-cancer activity.

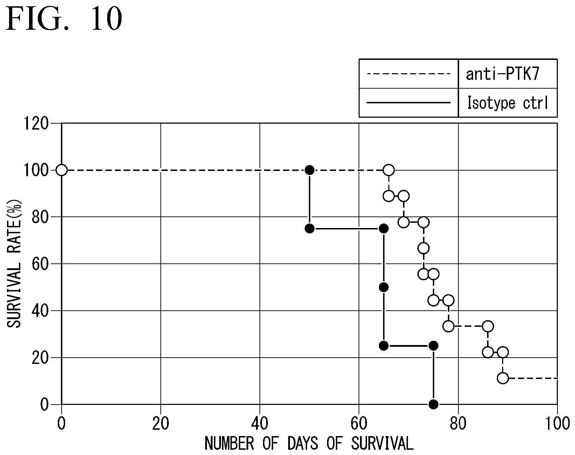

FIG. 10 is a graph showing the results obtained by observing the survival rate of the NOG mouse transplanted with hematopoietic stem cells over time. In FIG. 10, the vertical axis indicates the survival rate (%), and the horizontal axis indicates the number of days of survival (days). In addition, "anti-PTK7" indicates the results obtained by administering the #19 antibody, and "isotype ctrl" indicates the results obtained by administering an isotype control antibody.

As a result, as shown in FIG. 10, the mice in the #19 antibody administration group showed an extension of the survival period. This result indicates that the #19 antibody has an anti-cancer activity.

### INDUSTRIAL APPLICABILITY

According to the present embodiment, it is possible to provide an antibody against human PTK7 or a fragment thereof.

## Claims

1. An antibody or a fragment thereof, in which complementarity determining regions (CDR-H1 to CDR-H3) of a heavy chain variable region (V_{H} region) include each of amino acid sequences set forth in SEQ ID NOs: 1 to 3, and complementarity determining regions (CDR-L1 to CDR-L3) of a light chain variable region (V_{L} region) include each of amino acid sequences set forth in SEQ ID NOs: 4 to 6.

2. The antibody or the fragment thereof according to Claim 1,
wherein CDR-H1 to CDR-H3 of the V_{H} region consist of each of the amino acid sequences set forth in SEQ ID NOs: 1 to 3, and
CDR-L1 to CDR-L3 of the V_{L} region consist of each of the amino acid sequences set forth in SEQ ID NOs: 4 to 6 in no particular order.

3. The antibody or the fragment thereof according to Claim 1,
wherein the V_{II} region includes an amino acid sequence set forth in SEQ ID NO: 7, and CDR-H1 to CDR-H3 in the V_{II} region have each of an amino acid sequence from a position 50 to a position 54, an amino acid sequence from a position 69 to a position 85, and an amino acid sequence from a position 118 to a position 122 in the amino acid sequence set forth in SEQ ID NO: 7, and
the V_{L} region includes an amino acid sequence set forth in SEQ ID NO: 8, and CDR-L1 to CDR-L3 in the V_{L} region have each of an amino acid sequence from a position 49 to a position 59, an amino acid sequence from a position 75 to a position 81, and an amino acid sequence from a position 114 to a position 122 in the amino acid sequence set forth in SEQ ID NO: 8.

4. The antibody or the fragment thereof according to Claim 3,
wherein the V_{H} region consists of the amino acid sequence set forth in SEQ ID NO: 7, and
the V_{L} region consists of the amino acid sequence set forth in SEQ ID NO: 8.

5. The antibody or the fragment thereof according to Claim 4,
wherein a framework region (FR_{H}) in the V_{H} region has at least 80% sequence identity to FR_{II} in the continuous amino acid sequence set forth in SEQ ID NO: 7, and
a framework region (FR_{L}) in the V_{L} region has at least 80% sequence identity to FR_{L} in the continuous amino acid sequence set forth in SEQ ID NO: 8.

6. The antibody or the fragment thereof according to any one of Claims 1 to 5,
wherein the antibody or the fragment thereof binds to human PTK7.

7. An antibody or a fragment thereof, which binds to an epitope including an amino acid sequence from a position 310 to a position 331 and an amino acid sequence from a position 104 to a position 113 in an amino acid sequence set forth in any one of SEQ ID NOs: 9 to 14.

8. The antibody or the fragment thereof according to Claim 7,
wherein the epitope consists of an amino acid sequence from a position 310 to a position 331 in the amino acid sequence set forth in any one of SEQ ID NOs: 9 to 14.

9. The antibody or the fragment thereof according to Claim 7,
wherein the epitope consists of an amino acid sequence from a position 104 to a position 113 in the amino acid sequence set forth in any one of SEQ ID NOs: 9 to 14.

10. The antibody or the fragment thereof according to Claim 7,
wherein the antibody or the fragment thereof binds to an epitope including an amino acid sequence from a position 310 to a position 331 and an amino acid sequence from a position 104 to a position 113 in an amino acid sequence set forth in SEQ ID NO: 11.

11. The antibody or the fragment thereof according to Claim 7,
wherein the epitope consists of an amino acid sequence from a position 310 to a position 331 in an amino acid sequence set forth in SEQ ID NO: 11.

12. The antibody or the fragment thereof according to Claim 7,
wherein the epitope consists of an amino acid sequence from a position 104 to a position 113 in an amino acid sequence set forth in SEQ ID NO: 11.

13. An antibody or a fragment thereof, which competes with the antibody or the fragment thereof according to any one of Claims 1 to 5, when being bound to human PTK7.

14. The antibody or the fragment thereof according to Claim 13,
wherein the human PTK7 is a protein consisting of an amino acid sequence set forth in any one of SEQ ID NOs: 9 to 14.

15. The antibody or the fragment thereof according to Claim 14,
wherein the human PTK7 is a protein consisting of an amino acid sequence set forth in SEQ ID NO: 11.

16. The antibody or the fragment thereof according to any one of Claims 1 to 5 and 7 to 12,
wherein the antibody or the fragment thereof is a chimeric antibody, a humanized antibody, or a human antibody.

17. An anti-cancer agent comprising, as an active ingredient:
the antibody or the fragment thereof according to any one of Claims 1 to 5 and 7 to 12.

18. An anti-cancer pharmaceutical composition comprising:
the anti-cancer agent according to Claim 17; and
a pharmaceutically acceptable carrier.
